# EUROPÄISCHE PATENTSCHRIFT

(11) **EP 1 262 788 B1**
(45) Veröffentlichungstag und Bekanntmachung des Hinweises auf die Patenterteilung: **29.08.2007**
(21) Anmeldenummer: 02011096.1
(22) Anmeldetag: 17.05.2002
(51) Int. Cl.: G01R 33/385

(54) **Magnetresonanzgerät mit einer verfahrbaren Gradientenspuleneinheit**
MRI apparatus with a set of movable gradient coils
Appareil d'IRM avec un ensemble déplaçable de bobines de gradients

(30) Priorität: 30.05.2001 DE 10126337; 07.08.2001 DE 10138712
(43) Veröffentlichungstag der Anmeldung: 04.12.2002
(73) Patentinhaber: SIEMENS AKTIENGESELLSCHAFT, 80333 München (DE)
(72) Erfinder: Schaaf, Michael, 91097 Oberreichenbach (DE)

(56) Entgegenhaltungen:
- EP-A- 0 690 313
- US-A- 5 185 576
- US-A- 5 304 933

## Beschreibung

Die Erfindung betrifft ein Magnetresonanzgerät.

Die Magnetresonanztechnik ist eine bekannte Technik zum Gewinnen von Bildern eines Körperinneren eines Untersuchungsobjekts. Dabei werden in einem Magnetresonanzgerät einem statischen Grundmagnetfeld, das von einem Grundfeldmagneten erzeugt wird, schnell geschaltete Gradientenfelder überlagert, die von einem Gradientensystem erzeugt werden. Ferner umfasst das Magnetresonanzgerät ein Hochfrequenzsystem, das zum Auslösen von Magnetresonanzsignalen Hochfrequenzsignale in das Untersuchungsobjekt einstrahlt, und die ausgelösten Magnetresonanzsignale aufnimmt, auf deren Basis Magnetresonanzbilder erstellt werden.

Das Magnetresonanzgerät weist ein Abbildungsvolumen auf, in dem ein abzubildender Bereich des Untersuchungsobjekts zum Erstellen von Magnetresonanzbildern des abzubildenden Bereichs zu positionieren ist. Dazu umfasst das Magnetresonanzgerät eine wenigstens in einer Richtung verfahrbare Lagerungsvorrichtung, auf der das Untersuchungsobjekt gelagert werden kann. Dabei ist durch ein Verfahren der verfahrbaren Lagerungsvorrichtung samt dem darauf gelagerten Untersuchungsobjekt das Positionieren des abzubildenden Bereichs im Abbildungsvolumen möglich.

Beispielsweise aus der US 5,185,576 ist eine sogenannte lokale Gradientenspuleneinheit bekannt, die mit einer lokalen Hochfrequenzantenne kombiniert ist. Dabei ist die lokale Gradientenspuleneinheit mit integrierter lokaler Hochfrequenzantenne für einen speziellen Bereich des Untersuchungsobjekts, beispielsweise dem Kopf eines Patienten, ausgebildet. Dadurch ist die lokale Gradientenspuleneinheit gegenüber dem fest eingebauten Gradientenspulensystem mit kleineren Abmessungen ausführbar, was unter anderem hinsichtlich erzielbaren Gradientenstärken und Leistungsanforderungen an einen die Gradientenspuleneinheit speisenden Gradientenverstärker Vorteile bringt. Die lokale Gradientenspuleneinheit mit integrierter lokaler Hochfrequenzantenne ist dabei derart auf der Lagerungsvorrichtung befestigbar, dass sich die lokale Gradientenspuleneinheit auch bei Betrieb des Magnetresonanzgeräts und den dabei auf sie wirkenden Kräften nicht gegen die Lagerungsvorrichtung bewegt.

Aus US 5,304,933 A ist eine lokale Gradientenspule bekannt, deren Ausgestaltung es erlaubt, in Verbindung mit Stereotaxie-Geräten verwendet zu werden. Dazu ist es nötig, die lokale Gradientenspule in eine bestimmte Position in Bezug auf einen Stereotaxie-Rahmen zu bringen. Dies wird in axialer Richtung durch Stützen erreicht, die die lokale Gradientenspule gegen einen Tisch, an dem auch der Stereotaxie-Rahmen angebracht ist, abstützen.

Bei einigen Ausführungsformen von lokalen Gradientenspuleneinheiten dauert das bewegungsverhindernde sichere Befestigen der lokalen Gradientenspuleneinheit im Magnetresonanzgerät und auch umgekehrt das anschließende Wiederherauslösen der lokalen Gradientenspuleneinheit jeweils bis zu Stunden. Dabei ist bei manchen Ausführungsformen eine Ganzkörperantenne des Magnetresonanzgeräts aus- und wieder einzubauen. Unter anderem vorausgehend beschriebene lange Rüstzeiten bedingen eine geringe Bedienfreundlichkeit, so dass bisher lokale Gradientenspuleneinheiten fast ausschließlich im Forschungsbetrieb und nicht in der klinischen Routine eingesetzt werden.

Eine Aufgabe der Erfindung ist es, ein Magnetresonanzgerät zu schaffen, bei dem die Verwendung einer lokal einsetzbaren Gradientenspuleneinheit unter anderem ein hohes Maß an Bedienfreundlichkeit aufweist.

Die Aufgabe wird erfindungsgemäß durch den Gegenstand des Anspruchs 1 gelöst. Vorteilhafte Ausgestaltungen sind in den Unteransprüchen beschrieben.

Durch Anordnungen entsprechend dem Anspruch 1 wird ein hohes Maß an Bedienerfreundlichkeit, verbunden mit sehr kurzen Rüstzeiten, zum Einsatzbereitmachen der Gradientenspulen erzielt. Ein Ein- und Ausbauen einer Ganzkörperantenne zum Einsatzbereitmachen der Gradientenspuleneinheit entfällt vollständig.

In einer vorteilhaften Ausgestaltung umfasst das Magnetresonanzgerät ein fest eingebautes Gradientenspulensystem und die Mittel sind derart ausgebildet, dass die verfahrbare Gradientenspuleneinheit zum Fixieren gegen das Gradientenspulensystem abgestützt wird. Dadurch werden die von der verfahrbaren Gradientenspuleneinheit ausgehenden Kräfte direkt auf das fest eingebaute Gradientenspulensystem übertragen, das von Hause aus besser dazu geeignet ist, vorgenannte Kräfte aufzunehmen als beispielsweise eine Ganzkörperantenne, die zu diesem Zwecke zusätzlich versteift werden müsste. Des Weiteren wird dadurch die Ganzkörperantenne nicht durch mögliche Bewegungen der verfahrbaren Gradientenspuleneinheit belastet.

In einer vorteilhaften Ausgestaltung umfasst das Magnetresonanzgerät eine verfahrbare Lagerungsvorrichtung, die auf einer Führungsvorrichtung des Magnetresonanzgeräts verfahrbar ist, und die verfahrbare Gradientenspuleneinheit ist auf der gleichen Führungsvorrichtung verfahrbar ausgebildet. Dabei weist in einer Ausführungsform der Untersuchungsraum wenigstens zwei gegenüberliegende Öffnungen auf, so dass die Lagerungsvorrichtung von der einen Öffnung her und die verfahrbare Gradientenspuleneinheit von der anderen Öffnung her in den Untersuchungsraum hinein verfahren werden können. Dadurch eröffnet sich eine zeiteffiziente Nutzung des Magnetresonanzgeräts, bei der während dem Verfahren der Gradientenspuleneinheit das Untersuchungsobjekt, beispielsweise ein Patient, auf der Lagerungsvorrichtung gelagert wird. Nach dem Lagern des Patienten wird sodann die Lagerungsvorrichtung samt den darauf gelagerten Patienten in den Untersuchungsraum eingefahren, wo die Gradientenspuleneinheit bereits richtig positioniert und fixiert wartet.

Weitere Vorteile, Merkmale und Einzelheiten der Erfindung ergeben sich aus den im Folgenden beschriebenen Ausführungsbeispielen anhand der Zeichnung. Dabei zeigen:
Figur 1 einen Längsschnitt durch eine obere Hälfte eines Magnetresonanzgeräts mit einer verfahrbaren Gradientenspuleneinheit,
Figur 2 einen vergrößert dargestellten Ausschnitt aus Figur 1,
Figur 3 einen Querschnitt durch eine in Figur 1 dargestellte Ganzkörperantenne inklusive einer verfahrbaren Lagerungsvorrichtung und
Figur 4 einen vergrößert dargestellten Ausschnitt aus Figur 3.

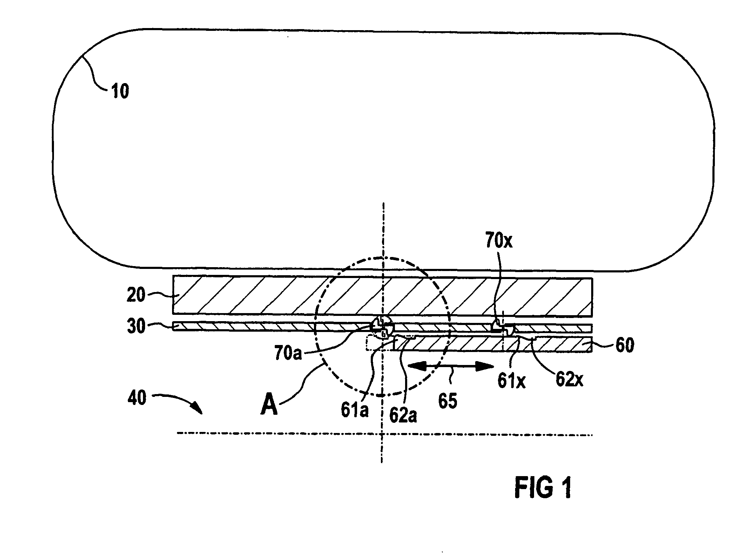

Die Figur 1 zeigt als ein Ausführungsbeispiel der Erfindung einen Längsschnitt durch eine obere Hälfte eines Magnetresonanzgeräts mit einer verfahrbaren Gradientenspuleneinheit 60. Das Magnetresonanzgerät umfasst zum Erzeugen eines statischen Grundmagnetfeldes einen im Wesentlichen hohlzylinderförmigen supraleitenden Grundfeldmagneten 10. In der Höhlung des Grundfeldmagneten 10 ist ein ebenfalls im Wesentlichen hohlzylinderförmiges Gradientenspulensystem 20 zum Erzeugen von Gradientenfeldern fest eingebaut. Dabei umfasst das fest eingebaute Gradientenspulensystem 20 drei Gradientenspulen, zu den Gradientenspulen zugehörige Abschirmspulen, Kühl- und Shim-Einrichtungen. In der Höhlung des Gradientenspulensystems 20 ist wiederum eine im Wesentlichen hohlzylinderförmige Ganzkörperantenne 30 zum Senden von Hochfrequenzsignalen und zum Empfangen von Magnetresonanzsignalen fest eingebaut, wobei eine Höhlung der Ganzkörperantenne 30 im Wesentlichen einen Untersuchungsraum 40 zum darin Lagern wenigstens eines Bereichs eines Untersuchungsobjekts, beispielsweise eines Patienten, definiert. Die verfahrbare Gradientenspuleneinheit 60 ist wenigstens in einen Teil des Untersuchungsraums 40 hinein verfahrbar. Dabei umfasst die Gradientenspuleneinheit 60 zum Erzeugen von Gradientenfeldern wenigstens eine bis hinauf zu drei Gradientenspulen und je nach Einsatzerfordernissen gegebenenfalls zu den Gradientenspulen zugehörige Abschirmspulen, Kühl- und Shim-Einrichtungen und/oder ist mit einer lokalen Hochfrequenzantenne kombiniert.

Die Ganzkörperantenne 30 ist als ein Träger für Mittel ausgebildet, mit denen die verfahrbare Gradientenspuleneinheit 60 wenigstens in einer Stellung in radialer Richtung fixierbar ist. Die Mittel umfassen dabei wenigstens sechs Fixiervorrichtungen 70a, 70b, 70c und 70x, die je um eine in der Ganzkörperantenne 30 angeordnete Drehachse 75a und 75c schwenkbar sind. Dabei sind drei der Fixiervorrichtungen 70a, 70b und 70c auf einer ersten und die weiteren drei Fixiervorrichtungen 70x auf einer zweiten Umfangslinie der Ganzkörperantenne 30 über den Umfang gleichmäßig verteilt angeordnet.

Die Fixiervorrichtungen 70a, 70b, 70c und 70x und die verfahrbare Gradientenspuleneinheit 60 sind derart ausgebildet, dass bei einem Verfahren der Gradientenspuleneinheit 60 gemäß der mit einem Doppelpfeil gekennzeichneten Verfahrrichtung 65 von rechts in den Untersuchungsraum 40 hinein die Fixiervorrichtungen 70a, 70b, 70c und 70x durch den Verfahrvorgang der Gradientenspuleneinheit 60 automatisch betätigt werden und gleichzeitig eine mit gestrichelten Linien dargestellte Endposition der verfahrbaren Gradientenspuleneinheit 60 definieren. Dazu weist die Gradientenspuleneinheit 60 auf ihrer Außenseite an Stellen, die entsprechend einer Anordnung der Fixiervorrichtungen 70a, 70b, 70c und 70x angeordnet sind, jeweils eine nasenartige Erhebung 61a und 61x in Verbindung mit einer Vertiefung 62a und 62x auf. Damit die komplett aus dem Untersuchungsraum 40 ausgefahrene Gradientenspuleneinheit 60 von rechts ordnungsgemäß in den Untersuchungsraum 40 bis zur Endposition eingefahren werden kann, sind die auf der linken Seite der Gradientenspuleneinheit 60 angeordneten Erhebungen 61a und Vertiefungen 62a, beispielsweise mittelpunktsnäher ausgebildet als die im Bereich der Mitte der Gradientenspuleneinheit 60 angeordneten Erhebungen 61x und Vertiefungen 62x. Dadurch werden beim Einfahren von rechts her nicht gleich durch die Erhebungen 61a und Vertiefungen 62a die Fixiervorrichtungen 70x betätigt, sondern können an diesen vorbei bewegt werden. Dies setzt selbstverständlich auch eine entsprechende Anordnung bzw. Ausbildung der Fixiervorrichtungen 70a, 70b, 70c und 70x voraus.

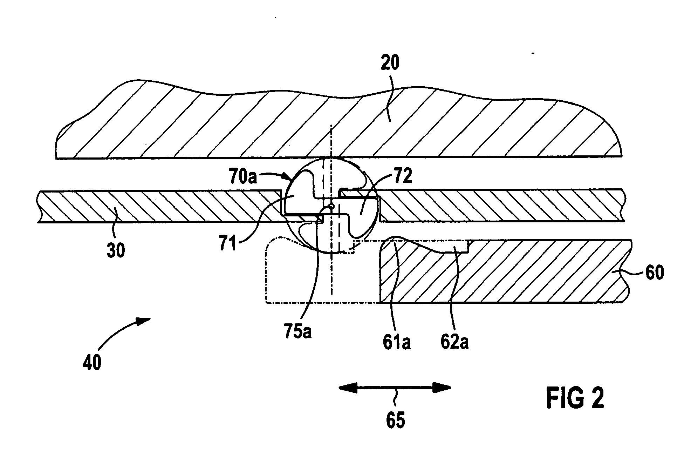

Eine genauere Beschreibung des Aufbaus einer der Fixiervorrichtungen 70a, 70b, 70c und 70x und deren Wirkung im Zusammenspiel mit der verfahrbaren Gradientenspuleneinheit 60 erfolgt anhand der Figur 2, die eine vergrößerte Darstellung des in der Figur 1 mit A bezeichneten Ausschnittes zeigt. Die Fixiervorrichtung 70a ist um eine Drehachse 75a schwenkbar in der Ganzkörperantenne 30 gelagert. Die Fixiervorrichtung 70a weist dabei eine bezüglich der Drehachse 75a symmetrische Formgebung, einen ersten und einen zweiten Nocken 71 und 72 auf, wobei der erste Nocken 71, beispielsweise durch Ausbildung aus einem Material hoher Dichte, ein größeres Gewicht als der zweite Nocken 72 aufweist, so dass sich automatisch bei nicht oder nicht vollständig eingefahrener Gradientenspuleneinheit 60 die mit durchgezogenen Linien dargestellte Lage der Fixiervorrichtung 70a einstellt. Bei einem Einfahren der verfahrbaren Gradientenspuleneinheit 60 von rechts in den Untersuchungsraum 40 nimmt die Erhebung 61a der Gradientenspuleneinheit 60 den zweiten Nocken 72 mit und versetzt die Fixiervorrichtung 70a um deren Drehachse 75a in eine Drehbewegung, bis der erste Nocken 71 in der mit gestrichelten Linien angedeuteten Stellung gegen das fest eingebaute Gradientenspulensystem 20 drückt. Sodann ist die verfahrbare Gradientenspuleneinheit 60 in radialer Richtung gegen das fest eingebaute Gradientenspulensystem 20 arretiert.

Das fest eingebaute Gradientenspulensystem 20 ist aufgrund seines Gießharzvergusses, seiner Ausbildung als Hohlzylinder großer Wandstärke und seiner Befestigung im Grundfeldmagneten 10 von Hause aus gut geeignet, die Druckbelastungen durch die Fixiervorrichtungen 70a, 70b, 70c und 70x aufzunehmen. Für ein Entfernen der verfahrbaren Gradientenspuleneinheit 60 aus dem Untersuchungsraum 40 ist die Gradientenspuleneinheit 60 in Verfahrrichtung 65 nach rechts zu bewegen. Ein Verfahren der Gradientenspuleneinheit 60 kann dabei sowohl von Hand als auch unter Zuhilfenahme eines motorischen Antriebs erfolgen. Die für eine Versorgung der Gradientenspuleneinheit 60 notwendigen elektrischen Anschlussleitungen und gegebenenfalls Kühlversorgungsleitungen sind der Gradientenspuleneinheit 60 dabei von rechts zugeführt.

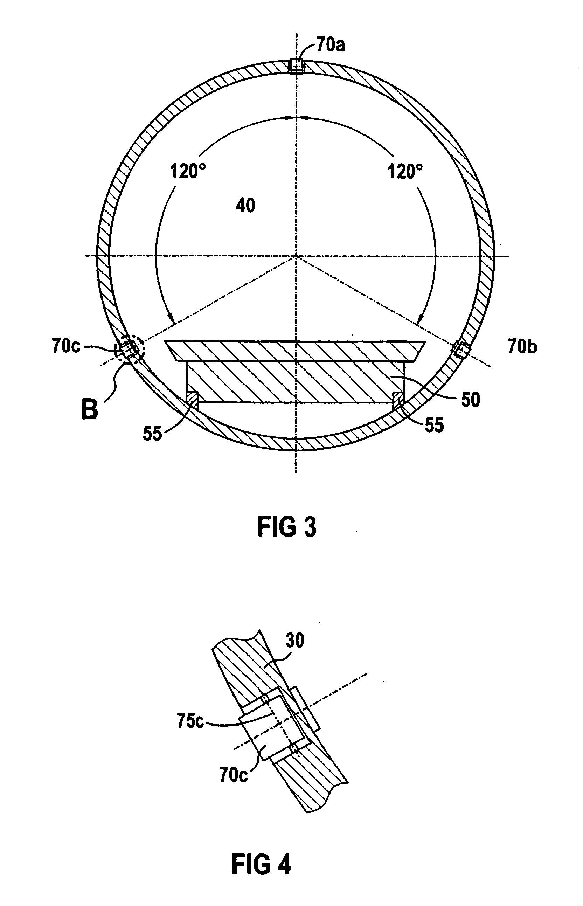

Die Figur 3 zeigt einen Querschnitt durch die Ganzkörperantenne 30 der Figur 1 im Bereich der Fixiervorrichtungen 70a, 70b und 70c inklusive einer verfahrbaren Lagerungsvorrichtung 50. In Figur 3 ist die bereits bei der Figur 1 beschriebene Gleichverteilung der drei Fixiervorrichtungen 70a, 70b und 70c über den Umfang mit 120°-Bogenpfeilen veranschaulicht. Mit der verfahrbaren Lagerungsvorrichtung 50 ist es möglich, das auf der Lagerungsvorrichtung 50 gelagerte Untersuchungsobjekt, beispielsweise den Patienten, bezüglich des in Figur 1 dargestellten Längsschnitts von links in den Untersuchungsraum 40 einzubringen. Dabei ist die Lagerungsvorrichtung 50 auf einem Führungssystem 55 verfahrbar gelagert. In einer Ausführungsform ist dabei auch die verfahrbare Gradientenspuleneinheit 60 auf dem gleichen Führungssystem 55 verfahrbar ausgebildet, was insbesondere bei einem Gewicht der Gradientenspuleneinheit 60 unproblematisch ist, das in etwa kleiner einem zulässigen Belastungsgewicht der Lagerungsvorrichtung 50 ist. Erst bei einem Gewicht der Gradientenspuleneinheit 60 von größer in etwa 200 kg sind besondere Maßnahmen vorzusehen.

Die Figur 4 zeigt schließlich in einer Vergrößerung den in Figur 3 mit B gekennzeichneten Ausschnitt. Mit der Figur 4 wird die Ausbildung und Anordnung der um die Drehachse 75c schwenkbar gelagerten Fixiervorrichtung 70c in der Ganzkörperantenne 30 nochmals verdeutlicht.

Schließlich sind auch bestehende Magnetresonanzgeräte in einfacher und kostengünstiger Weise zu einem Magnetresonanzgerät mit verfahrbarer Gradientenspuleneinheit 60 entsprechend Figur 1 umrüstbar. Dazu ist lediglich eine Ganzkörperantenne des bestehenden Magnetresonanzgeräts gegen eine vorausgehend beschriebene Ganzkörperantenne 30 mit Fixiervorrichtungen 70a, 70b, 70c und 70x zu tauschen und die verfahrbare Gradientenspuleneinheit 60 hinzuzufügen.

## Patentansprüche

1. Magnetresonanzgerät, beinhaltend folgende Merkmale:
- Einen Untersuchungsraum (40) zum darin Lagern wenigstens eines Bereichs eines Untersuchungsobjekts,
- eine in einer Verfahrrichtung (65) in den Untersuchungsraum (40) verfahrbare Gradientenspuleneinheit (60),
- eine den Untersuchungsraum (40) umgebende Komponente des Magnetresonanzgeräts,
**dadurch gekennzeichnet, dass**
- zwischen dem Untersuchungsraum (40) und der Komponente Mittel (70a, 70b, 70c, 70x) angeordnet sind, mit denen die Gradientenspuleneinheit (60) zum Fixieren in wenigstens einer Stellung im Magnetresonanzgerät gegen die Komponente abstützbar ist, und dass
- eine zwischen dem Untersuchungsraum (40) und der Komponente angeordnete Ganzkörperantenne (30) derart ausgebildet ist, dass die Mittel (70a, 70b, 70c, 70x) durch die Ganzkörperantenne (30) hindurch abstützen.

2. Magnetresonanzgerät nach Anspruch 1, wobei die Komponente derart ausgebildet ist, dass die Gradientenspuleneinheit (60) dagegen abstützbar ist.

3. Magnetresonanzgerät nach einem der Ansprüche 1 oder 2, wobei die Komponente ein fest eingebautes Gradientenspulensystem (20) des Magnetresonanzgeräts umfasst.

4. Magnetresonanzgerät nach einem der Ansprüche 1 bis 3, wobei die Ganzkörperantenne (30) zum Abstützen der Gradientenspuleneinheit (60) gegen die Komponente wenigstens eine Öffnung aufweist.

5. Magnetresonanzgerät nach einem der Ansprüche 1 bis 4, wobei die Ganzkörperantenne (30) als ein Träger für die Mittel (70a, 70b, 70c, 70x) ausgebildet ist.

6. Magnetresonanzgerät nach einem der Ansprüche 1 bis 5, wobei die Mittel (70a, 70b, 70c, 70x) und die Gradientenspuleneinheit (60) derart ausgebildet sind, dass durch ein Verfahren der Gradientenspuleneinheit (60) die Mittel (70a, 70b, 70c, 70x) betätigbar sind.

7. Magnetresonanzgerät nach einem der Ansprüche 1 bis 6, wobei die Mittel (70a, 70b, 70c, 70x) und die Gradientenspuleneinheit (60) derart ausgebildet sind, dass die Mittel (70a, 70b, 70c, 70x) eine Endposition der Gradientenspuleneinheit (60) in Verfahrrichtung (65) bewirken.

8. Magnetresonanzgerät nach einem der Ansprüche 1 bis 7, wobei die Mittel (70a, 70b, 70c, 70x) wenigstens eine um eine Drehachse (75a, 75c) schwenkbare Fixiervorrichtung (70a, 70b, 70c, 70x) umfassen.

9. Magnetresonanzgerät nach Anspruch 8, wobei die Fixiervorrichtung (70a, 70b, 70c, 70x) bezüglich der Drehachse (75a, 75c) symmetrisch ausgebildet ist.

10. Magnetresonanzgerät nach einem der Ansprüche 8 oder 9, wobei die Fixiervorrichtung (70a, 70b, 70c, 70x) beiderseits der Drehachse (75a, 75c) je einen Nocken (71, 72) aufweist.

11. Magnetresonanzgerät nach Anspruch 10, wobei einer der Nocken (71) ein größeres Gewicht als der andere der Nocken (72) aufweist.

12. Magnetresonanzgerät nach einem der Ansprüche 8 bis 11, wobei wenigstens drei Fixiervorrichtungen (70a, 70b, 70c) entlang eines Umfangs des Untersuchungsraums (40) in etwa gleichverteilt angeordnet sind.

13. Magnetresonanzgerät nach einem der Ansprüche 1 bis 12, wobei das Magnetresonanzgerät eine verfahrbare Lagerungsvorrichtung (50) und eine Führungsvorrichtung (55), auf der die Lagerungsvorrichtung (50) verfahrbar ist, umfasst und die Gradientenspuleneinheit (60) auf der Führungsvorrichtung (55) verfahrbar ausgebildet ist.

14. Magnetresonanzgerät nach einem der Ansprüche 1 bis 13, wobei der Untersuchungsraum (40) wenigstens zwei gegenüberliegende Öffnungen aufweist und eine verfahrbare Lagerungsvorrichtung (50) von der einen Öffnung her und die Gradientenspuleneinheit (60) von der anderen Öffnung her in den Untersuchungsraum (40) hinein verfahrbar sind.

15. Magnetresonanzgerät nach einem der Ansprüche 6 bis 14, wobei eine Oberfläche der Gradientenspuleneinheit (60) zum Betätigen der Mittel (70a, 70b, 70c, 70x) wenigstens eine nasenartige Erhebung (61a, 61x) in Verbindung mit einer Vertiefung (62a, 62x) aufweist.

## Claims

1. Magnetic resonance apparatus having the following features :
- an examination space (40) in which at least a region of an examination object can be supported,
- a gradient coil unit (60) that can be displaced into the examination space (40) in a displacement direction (65),
- a component of the magnetic resonance apparatus surrounding the examination space (40),
**characterised in that**
- means (70a, 70b, 70c, 70x) are arranged between the examination space (40) and the component, with which the gradient coil unit (60) can be supported against the component to fix said gradient coil unit (60) in at least one position in the magnetic resonance apparatus and that
- a whole-body antenna (30) arranged between the examination space (40) and the component is configured such that the means (70a, 70b, 70c, 70x) support through the whole-body antenna (30).

2. Magnetic resonance apparatus according to claim 1, wherein said component is configured such that the gradient coil unit (60) can be supported against it.

3. Magnetic resonance apparatus according to one of claims 1 or 2, wherein the component comprises a permanently installed gradient coil system (20) of the magnetic resonance apparatus.

4. Magnetic resonance apparatus according to one of claims 1 to 3, wherein the whole body antenna (30) has at least one opening to support the gradient coil unit (60) against the component.

5. Magnetic resonance apparatus according to one of claims 1 to 4, wherein the whole body antenna (30) is configured as a carrier for the means (70a, 70b, 70c, 70x).

6. Magnetic resonance apparatus according to one of claims 1 to 5, wherein the means (70a, 70b, 70c, 70x) and the gradient coil unit (60) are configured such that the means (70a, 70b, 70c, 70x) can be actuated by displacement of the gradient coil unit (60).

7. Magnetic resonance apparatus according to one of claims 1 to 6, wherein the means (70a, 70b, 70c, 70x) and the gradient coil unit (60) are configured such that the means (70a, 70b, 70c, 70x) effect an end position for the gradient coil unit (60) in the displacement direction (65).

8. Magnetic resonance apparatus according to one of claims 1 to 7, wherein the means (70a, 70b, 70c, 70x) comprise at least one fixing mechanism (70a, 70b, 70c, 70x) that can be pivoted around a rotational axis (75a, 75c).

9. Magnetic resonance apparatus according to claim 8, wherein the fixing mechanism (70a, 70b, 70c, 70x) is configured as symmetrical relative to the rotational axis (75a, 75c).

10. Magnetic resonance apparatus according to one of claims 8 or 9, wherein the fixing mechanism (70a, 70b, 70c, 70x) comprises one cam (71, 72) respectively on both sides of the rotational axis (75a, 75c).

11. Magnetic resonance apparatus according to claim 10, wherein one of the cams (71) has a greater weight than the other of the cams (72).

12. Magnetic resonance apparatus according to one of claims 8 to 11, wherein at least three fixing mechanisms (70a, 70b, 70c, 70x) are arranged in a roughly, uniformly distributed manner along, a circumference of the examination space (40).

13. Magnetic resonance apparatus according to one of claims 1 to 12, wherein the magnetic resonance apparatus comprises a displaceable support mechanism (50) and a guide mechanism (55), on which the support mechanism (50) can be displaced and the gradient coil unit (60) is configured such that it can be displaced on the guide mechanism (55).

14. Magnetic resonance apparatus according to one of claims 1 to 13, wherein the examination space has at least two opposite openings and a displaceable support mechanism (50) can be displaced from one opening and the gradient coil unit (60) can be displaced from the other opening into the examination space (40).

15. Magnetic resonance apparatus according to one of claims 6 to 14, wherein a top surface of the gradient coil unit (60) has at least one nose-like projection (61a, 61x) in combination with a recess (62a, 62x) to actuate the means (70a, 70b, 70c, 70x).

## Revendications

1. Appareil à résonance magnétique, comprenant les caractéristiques suivantes :
- une chambre d'examen (40) pour y loger au moins une partie d'un objet d'examen,
- une unité de bobines de gradients (60) déplaçable dans la chambre d'examen dans une direction de déplacement (65),
- un composant de l'appareil à résonance magnétique, entourant la chambre d'examen (40),
**caractérisé en ce qu'**
- entre la chambre d'examen (40) et le composant, des moyens (70a, 70b, 70c, 70x) sont disposés, au moyen desquels l'unité de bobines de gradients peut s'appuyer contre le composant pour sa fixation dans au moins une position dans l'appareil à résonance magnétique, et **en ce qu'**
- une antenne pour corps entier (30) disposée entre la chambre d'examen (40) et le composant est exécutée de telle manière que les moyens (70a, 70b, 70c, 70x) s'appuient en traversant l'antenne pour corps entier (30).

2. Appareil à résonance magnétique selon la revendication 1, le composant étant exécuté de telle manière que l'unité de bobines de gradients (60) peut s'appuyer contre lui.

3. Appareil à résonance magnétique selon l'une quelconque des revendications 1 ou 2, le composant comprenant un système de bobines de gradients (20), installé de manière fixe, de l'appareil à résonance magnétique.

4. Appareil à résonance magnétique selon l'une quelconque des revendications 1 à 3, l'antenne pour corps entier (30) présentant au moins une ouverture pour l'appui de l'unité de bobines de gradients (60) contre le composant.

5. Appareil à résonance magnétique selon l'une quelconque des revendications 1 à 4, l'antenne pour corps entier (30) étant exécutée en tant qu'un porteur pour les moyens (70a, 70b, 70c, 70x).

6. Appareil à résonance magnétique selon l'une quelconque des revendications 1 à 5, les moyens (70a, 70b, 70c, 70x) et l'unité de bobines de gradients (60) étant exécutés de telle manière que les moyens (70a, 70b, 70c, 70x) peuvent être actionnés au moyen d'un procédé de l'unité de bobines de gradients (60).

7. Appareil à résonance magnétique selon l'une quelconque des revendications 1 à 6, les moyens (70a, 70b, 70c, 70x) et l'unité de bobines de gradients (60) étant exécutés de telle manière que les moyens (70a, 70b, 70c, 70x) ont pour effet une position finale de l'unité de bobines de gradients (60) dans la direction de déplacement (65).

8. Appareil à résonance magnétique selon l'une quelconque des revendications 1 à 7, les moyens (70a, 70b, 70c, 70x) comprenant au moins un dispositif de fixation (70a, 70b, 70c, 70x) pivotant autour d'un axe de rotation (75a, 75c).

9. Appareil à résonance magnétique selon la revendication 8, le dispositif de fixation (70a, 70b, 70c, 70x) étant exécuté de manière symétrique par rapport à l'axe de rotation (75a, 75c) .

10. Appareil à résonance magnétique selon l'une quelconque des revendications 8 ou 9, le dispositif de fixation (70a, 70b, 70c, 70x) présentant un mentonnet (71, 72) de chaque côté de l'axe de rotation (75a, 75c).

11. Appareil à résonance magnétique selon la revendication 10, l'un des mentonnets (71) présentant un poids supérieur à celui de l'autre des mentonnets (72).

12. Appareil à résonance magnétique selon l'une quelconque des revendications 8 à 11, au moins trois dispositifs de fixation (70a, 70b, 70c) étant disposés le long d'une circonférence de la chambre d'examen (40) de manière à peu près uniformément répartie.

13. Appareil à résonance magnétique selon l'une quelconque des revendications 1 à 12, l'appareil à résonance magnétique comprenant un dispositif de logement (50) mobile et un dispositif de guidage (55), sur lequel le dispositif de logement (50) est déplaçable, et l'unité de bobines de gradients (60) étant exécutée de manière déplaçable sur le dispositif de guidage (55).

14. Appareil à résonance magnétique selon l'une quelconque des revendications 1 à 13, la chambre d'examen (40) présentant au moins deux ouvertures opposées et un dispositif de logement mobile (50) étant déplaçable dans la chambre d'examen (40) depuis l'une des ouvertures et l'unité de bobines de gradients (60) étant déplaçable dans la chambre d'examen (40) depuis l'autre ouverture.

15. Appareil à résonance magnétique selon l'une quelconque des revendications 6 à 14, une surface de l'unité de bobines de gradients (60) présentant au moins une élévation (61a, 61x) en forme d'ergot, en liaison avec un approfondissement (62a, 62x), pour l'actionnement des moyens (70a, 70b, 70c, 70x).
